# EUROPEAN PATENT APPLICATION

(11) **EP 2 660 742 A1**
(43) Date of publication of application: **06.11.2013**
(21) Application number: 12166349.6
(22) Date of filing: 02.05.2012
(51) Int. Cl.: G06F 19/00

(54) **Training apparatus**

(71) Applicant: Hocoma AG, 8604 Volketswil (CH)
(72) Inventor: Lawrence, Marc, 8620 Wetzikon (CH); Colombo, Gery, 8610 Uster (CH); Koll, Andreas, 8051 Zürich (CH); Keemink, Rogier, 8050 Zürich (CH)
(74) Representative: Sticht, Andreas

(57) **Abstract**

Training apparatuses and corresponding methods are disclosed, where a first range of movement of a person (10) and a second range of movement within the first range of movement or other movement-related parameter spaces of the person (10) is captured and stored in a storage. In some cases, feedback during training may be given depending on the first range of movement and/or the second range of movement.

## Description

This application relates to training apparatuses and corresponding methods performed using such training apparatuses. Such training apparatuses may for example be used for physiotherapy, for example physiotherapy of the back of a person.

Conventionally, physiotherapy is performed by trained physiotherapists who ensure that their patients perform physiologically correct movements to alleviate their respective problems, for example by training the back of the patient to alleviate back pain. In recent years, there has been an increasing development of training apparatuses which enable a person to perform physiotherapeutic exercises without supervision or at least without constant supervision by a physiotherapist. In such systems, typically a sensor arrangement is used to monitor movements of the person, e.g. patient using the system. One example for such a training apparatus is the Valedo System of Hocoma AG, the applicant of the present application. In this apparatus, inertial measurement units (IMUs) are attached to the back of a person for measuring movements thereof, and by moving the back the person can for example control elements displayed on a screen, for example a so-called Avatar, to perform specific exercises.

Generally, it is desirable to tailor the exercises to the person performing them in order to achieve good training effects and/or to document the training progress of the persons.

In view thereof, it is an object of the present invention to provide a training apparatus and a corresponding method which facilitates monitoring the progress of a person performing the training and/or better adapting the training to the person.

According to an aspect of the invention, a training apparatus as defined in claim 1 is provided. The dependent claims define further embodiments.

According to an aspect, a training apparatus is provided, comprising:
a sensor arrangement configured to capture a movement-related parameter, for example a movement, of a person,
a storage, and
an evaluation unit coupled to the sensor arrangement and the storage, wherein said evaluation unit is configured to capture a first movement-related parameter space of the person and to capture a second movement-related parameter space of the person at least partially within the first movement-related parameter space via the sensor arrangement, and to store the first movement-related parameter space and the second movement-related parameter space in said storage.

The first movement-related parameter space may for example be a first range of movement of the person, and the second movement-related parameter space may comprise a second movement range of the person within the first movement range. In this case, the first movement range may for example be an active or passive maximum movement range, and the second movement range may be a comfortable movement range, i.e. a movement range where the person is able to move without pain, or a desired exercise movement range, or any other movement range tailored to the training goal and to the person. In other embodiments, the first movement range may be a passive maximum movement range, i.e. a movement range where limbs or the like of the person are guided for example by a therapist, and the second movement range may be an active maximum movement range, i.e. a movement range in which a person is able to move a limb or other body part due to his or her own muscular efforts.

In other embodiments, the first movement-related parameter space may comprise a first isometric force, and the second movement-related parameter space may comprise a second isometric force smaller than the first isometric force. An isometric force, in this case, is a force exercised by a limb or other body part against for example an unmoving surface equipped with a pressure sensor or against any other object (for example other body part) which prevents a movement which would otherwise occur. For example, the first and second isometric forces may be gripping forces caused by flexing fingers of a hand of the person.

By storing such first and second movement-related parameter spaces, a training progress of the person may be documented. Moreover, training exercises may be based on the first movement-related parameter space and/or the second movement-related parameter space and therefore be adapted to the person.

The first movement-related parameter space and the second movement-related parameter space each may be a movement-related parameter space for a specific body part, for example a limb. Furthermore, the first movement-related parameter space and/or the second movement-related parameter space may be a three-dimensional space, e.g. a "movement space", or may be a parameter space related to a specific movement direction. A plurality of first and second movement-related parameter spaces may be captured for different movement directions and/or different body parts.

The capturing of the first movement-related parameter space and the second movement-related parameter space may in particular be done during an initialization phase prior to a training phase or between training phases.

In an embodiment, the device further comprises an output arrangement, for example a display, one or more loudspeakers or a combination thereof. In such an embodiment, the evaluation unit may be configured to capture, during a training phase, a movement-related parameter, e.g. a movement, of a person via the sensor arrangement and to output a feedback via the output arrangement based on the movement-related parameter. Such a feedback may for example be based on the first movement-related parameter space and/or the second movement-related parameter space. For example, the captured movement-related parameter during the training phase may be compared to the first movement-related parameter space and/or to the second movement-related parameter space to generate the feedback.

In some embodiments, the output arrangement may comprise a display, and the feedback may comprise a movable object, the movable object moving depending on the captured movement-related parameter of the person, and movement incentives prompting a person who performs the training to perform specific movements. The translation of the captured movements to movements of the movable object on the display, also referred to as mapping, may in some embodiments be made depending on the first range of movement and/or depending on the second range of movement. In other embodiments, the display of the movement incentives, for example their positioning, may be made depending on the first movement-related parameter space and/or depending on the second movement-related parameter space.

In some embodiments, furthermore the device may comprise an input arrangement allowing a user to input information. In some embodiments, the input arrangement may be configured to receive a sensitivity input from the user, and the mapping of movements of the person training to movements of the movable object may be made on the basis of the sensitivity input. The sensitivity input may in some embodiments be scaled depending on the first movement-related parameter space and/or depending on the second movement-related parameter space. In some embodiments, the feedback may be adjusted based on a comparison between the first and/or second movement-related parameter spaces as captured before a training compared with first and/or second movement-related parameter spaces which were stored in previous training sessions.

According to another aspect of the invention, a method is provided, comprising:
capturing a first movement-related parameter space of a person which e.g. is a first range of movement, and
capturing a second movement-related parameter space within the first movement-related parameter space, e.g. a second range of movement, and
storing the first and second movement-related parameter spaces.

In an embodiment, the method may further comprise providing feedback to the person during a training exercise, wherein the feedback may for example be based on the first movement-related parameter space, the second movement-related parameter space and/or a sensitivity input. The feedback and the sensitivity input may be as described above.

The above-mentioned features, embodiments and aspects may be combined with each other unless specifically noted otherwise.

Further embodiments will be described with reference to the attached drawings, wherein:
Fig. 1 is a block diagram of a training apparatus according to an embodiment;
Fig. 2 is a diagram showing a sensor arrangement usable in some embodiments;
Fig. 3 is a flowchart representing a method according to an embodiment of the present invention;
Fig. 4 is an example representation of elements shown on a display to illustrate some features of some embodiments of the present invention;
Figs. 5A and 5B are schematic diagrams illustrating movement ranges;
Figs. 6A and 6B are schematic representations showing sensor arrangements usable in some embodiments of the present invention; and
Fig. 7 is a schematic illustration of elements shown on a display to illustrate feedback usable in some embodiments of the present invention.

In the following, embodiments of the present invention will be described in detail with reference to the attached drawings. It should be noted that features of different embodiments may be combined with each other unless specifically noted otherwise. On the other hand, describing an embodiment with a plurality of features is not to be construed as indicating that all those features are necessary for practicing the invention, as other embodiments may comprise less features and/or alternative features.

In the following, a training apparatus and corresponding methods will be described. Such training apparatuses and methods may in particular be used for therapeutic purposes, e.g. physiotherapy or rehabilitation training, but may also be used for general training purposes, e.g. non-therapeutic purposes.

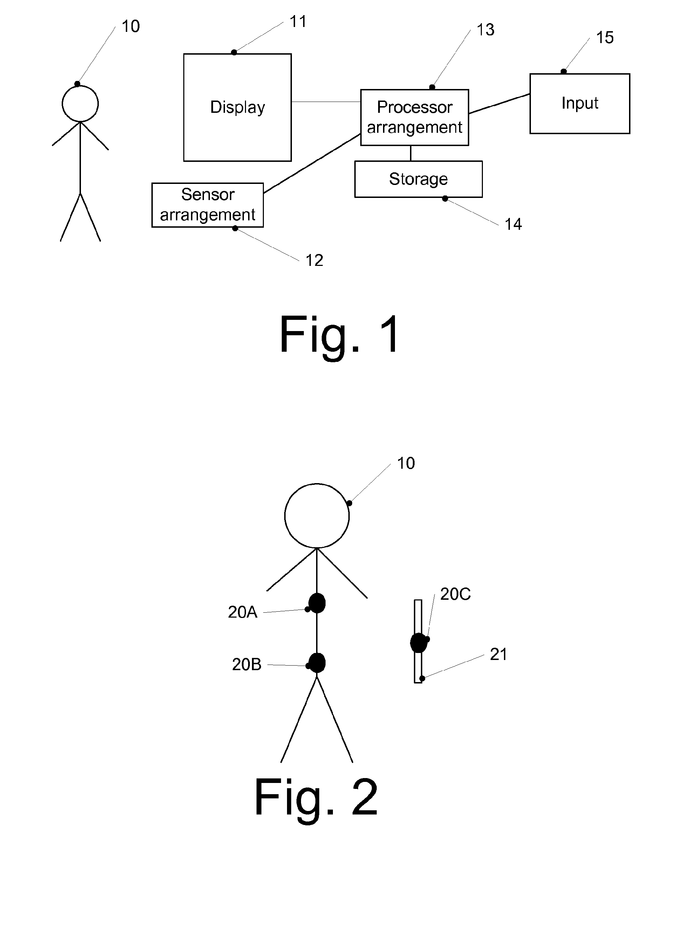

In Fig. 1, a schematic block diagram of a training apparatus according to an embodiment is shown.

The apparatus of Fig. 1 comprises a sensor arrangement 12 with which the apparatus may capture movements of a person 10, person 10 for example being a patient to be treated by physiotherapy or a person using the training apparatus for other purposes. In an embodiment described in the following in some more details, sensor arrangement 12 may comprise inertial measurement units (IMUs), i.e. sensors which comprise accelerometers, gyroscopes and/or similar devices to capture also small movements of person 10. Suitable IMUs are commercially available for example from Movea SA under the trademark MotionPod.

An example for such a sensor arrangement using IMUs is shown in Fig. 2. Here, two sensors 20A, 20B may be fixed to the back of a person 10, for example a sensor 20B at the lower back and a sensor 20A rather in the middle of the back of person 10 to capture movements of the back, in particular movements of a pelvic region and movements of an upper torso, independently from each other. A third sensor 20C fixed to a rod-like element 21 may be used for calibration purposes, for example to provide a reference frame (for example to determine the initial orientation of sensors 20A, 20B in a stationary reference coordinate system). Such an initialization via sensor 20C may be performed at the beginning of a training session. Sensors 20A-20C may communicate in a wireless manner, for example via Bluetooth, with the remaining training apparatus.

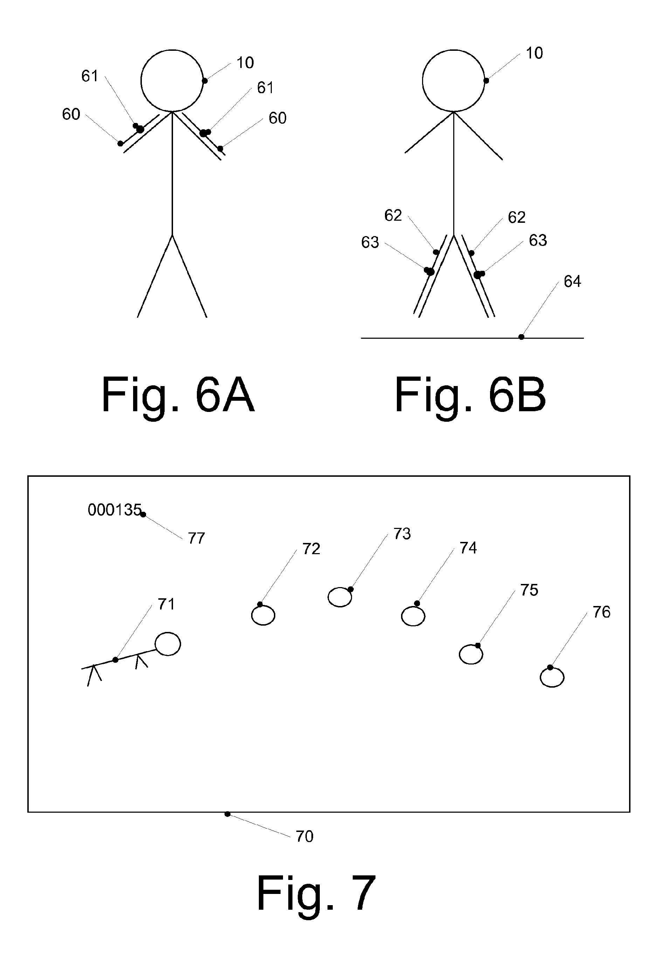

It should be noted that embodiments of the invention are not limited to the sensor arrangement shown in Fig. 2, and other types of sensors, some of which will be discussed later in some more detail with reference to Fig. 6, may be used. For example, in addition or alternatively to sensors which in some way are affixed to person 10, optical sensors, for example a 3D camera, may be used in some embodiments. Also, the number and/or positioning of sensors shown in Fig. 2 may be varied depending on the application.

Returning to Fig. 1, sensor arrangement 12 is coupled with a processor arrangement 13 serving as an evaluation unit to evaluate signals output by sensor arrangement 12. As already mentioned, the coupling between sensor arrangement 12 and processor arrangement 13 may be a wireless coupling. In other embodiments, a wire-based coupling may be used. Furthermore, processor arrangement 13 is coupled with a display 11, an input 15 and a storage 14. Also these couplings each may be wireless couplings or wire-based couplings. For example, display 11 may be coupled with processor arrangement 13 in a wireless manner to enable an arbitrary placement of display 11 independent of the rest of the apparatus such that person 10 may place display 11 such that it may be viewed during training exercises performed using the training apparatus of Fig. 1. Processor arrangement 13 may comprise one or more microprocessors configured to execute programs stored in storage 14. Storage 14 may comprise one or more different types of storage like RAM, ROM, flash memory, hard disks, and the like. Input 15 may comprise one or more input devices like keyboard, mouse or trackball. In some embodiments, display 11 may be a touch-sensitive display (so-called touchscreen) and therefore may also serve as input 15 or part thereof. In some embodiments, processor arrangement 13, storage 14 and input 15 may be implemented by a conventional personal computer (PC) which is programmed accordingly.

Processor arrangement 13 is configured to, for example by executing corresponding programs stored in storage 14, capture a first movement-related parameter space of person 10 e.g. corresponding to a first range of movement and a second movement-related parameter space of person 10 at least partially within the first movement-related parameter space, e.g. corresponding to a second range of movement, for example during an initialization phase preceding a training phase or also after a training phase, using sensor arrangement 12. To achieve this, the processor arrangement may instruct person 10 to perform a corresponding movement-related action and then capture the movement-related parameter space via sensor arrangement 12. Also, person 10 or another person may initiate the recording of the first and second movement-related parameter spaces via a user input using input 15. The first movement-related parameter space and the second movement-related parameter space in the embodiment of Fig. 1 are then stored in storage 14 for future reference.

In the following, first and second movement ranges will be used as example for first and second parameter spaces. The first range of movement may for example be a maximum range of movement, for example an active maximum range of movement, i.e. a range of movement through which a person can actively move a body part using his or her own muscular power, or a maximum passive range of movement, i.e. a range of movement through which a body part of the person may be moved for example by a physiotherapist without causing pain and/or injury to the person.

The second range of movement may for example be a comfortable range of movement, i.e. a range of movement where person 10 feels no pain, or an exercise range of movement, i.e. a range of movement which is to be used as a basis for a following training exercise, for example at a start of a following training exercise. In other embodiments, the first range of movement may be a passive maximum range of movement, and the second range of movement may be an active maximum range of movement.

The first and second range of movement each may be three-dimensional ranges, i.e. "movement spaces", within which a specific part of person 10, for example a limb or another body part, may move in, or may be a range of movement in a specific movement direction. In particular for the latter case also a plurality of first and second ranges for different movement directions may be recorded. Corresponding examples will be discussed later. The data of the sensor arrangement 12 is then translated into a coordinate system, for example a coordinate system of the person 10 or the body part moving. The first and second ranges of movement may then be stored in this coordinate system.

During training, processor arrangement 13 captures movements of person 10 via sensor arrangement 12 and outputs a feedback on display 11, which feedback may for example comprise instructions to person 10 regarding the training, a score or other evaluation of the training. The instructions may also take the form of a game-like environment where person 10, through his or her movements, controls a movable object (sometimes referred to as Avatar) to achieve certain goals. Examples for this will also be discussed later in greater detail.

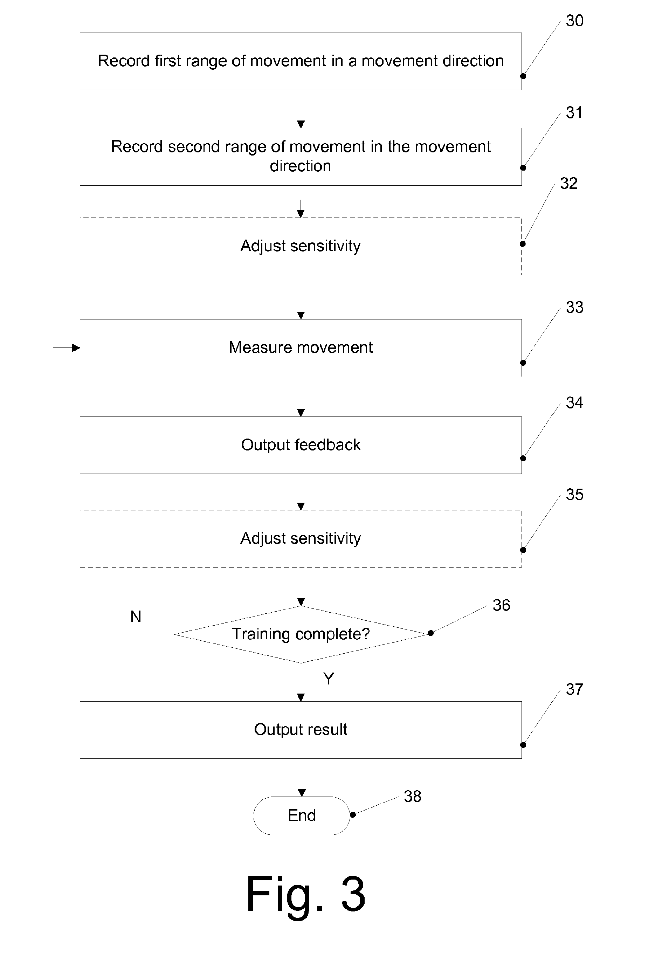

The above concepts and features will now be explained in some more detail with respect to Figs. 3-7. Fig. 3 shows a flowchart of a method according to an embodiment. The method of Fig. 3 may for example be implemented in the training apparatus of Fig. 1, for example by corresponding programming of the training apparatus. However, the embodiment of Fig. 3 may also be used independently from the embodiment of Fig. 1.

While the method of Fig. 3 is represented as a series of acts or events, it should be noted that the acts and events shown need not necessarily be performed in the order shown, but may also be performed in a different order, and/or some of the acts or events may be performed concurrently with each other. Moreover, in some embodiments some acts or events may be omitted entirely.

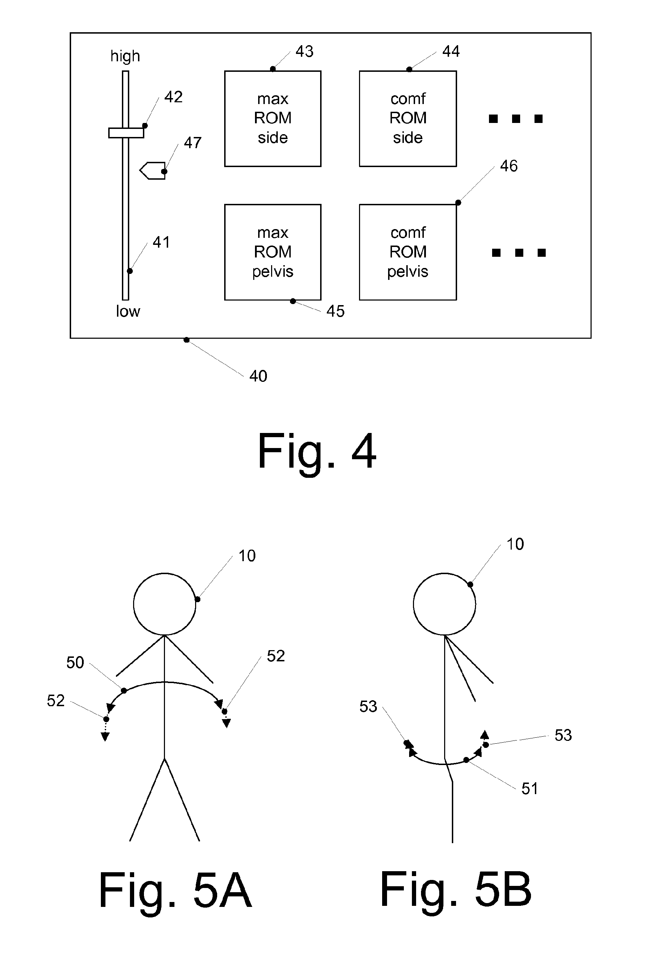

At 30, a first range of movement of a person, for example a specific limb or body part of a person, in a movement direction is recorded, i.e. captured and stored. At 31, a second range of movement of the person in the movement direction is recorded, for example a comfortable range or an exercise range as described above. Steps 30 and 31 may for example be performed during an initialization prior to training. At 30 and 31, also a plurality of first ranges and second ranges for movements in different movement directions may be recorded. Furthermore, additionally or alternatively first ranges and second ranges for three-dimensional movements, i.e. a movement comprising more than one movement direction, may be recorded, or, in other words, in this case the range of movement corresponds to a "movement space". Examples for the actions described with reference to 30 and 31 will now be explained with reference to Figs. 4 and 5.

As an example, in Figs. 4 and 5 the recording of a first range of movement and a second range of movement for two different movement directions, namely a side movement of a torso and a tilting movement of a pelvis of a person, is shown.

In Fig. 4, a schematic representation 40 of elements 41-46 shown on a display of a training apparatus, for example display 11 of Fig. 1, is shown. In particular, in Fig. 4, four buttons 43-46 are shown. In this example, when a user activates button 43, a maximum range of movement (ROM) of an upper body in a side direction is recorded (i.e. a person training performs the movement which is captured using appropriate sensors), and when activating button 44, a comfortable range of movement, i.e. a second range of the movement, in the same movement direction is recorded. Activation of buttons 43 and 44 may for example be effected by touching the buttons in case the display is a touchscreen or by clicking on the buttons for example with a mouse pointer.

To illustrate this, Fig. 5A shows person 10 with the corresponding ranges of movement. The sideway movement of the upper torso is indicated with arrows. In solid lines and designated 50, a comfortable range of movement or exercise range of movement, for example a range of movement without causing pain to person 10, is shown. Continuing with dashed lines and labeled 52, the maximum range of movement, for example the maximum range of movement person 10 can perform due to his or her own muscular power, is shown. When now button 43 in Fig. 4 is activated, person 10 performs the movement e.g. through the active maximum range of movement (50+52), the corresponding movement is captured by sensors, for example the sensors shown in Fig. 2, and then stored. Thereafter button 44 may be activated, and person 10 may perform a movement through the comfortable range (50 only). It should be noted that both the maximum range and the comfortable range may be asymmetric (different movement capabilities towards left and right side in the example of Fig. 5A) although depending on person 10 they may also be symmetric.

In a similar manner, with button 45 a maximum range of movement for a tilting of the pelvis is recorded (first range of movement), and with button 46 a corresponding comfortable range of movement (second range of movement) is recorded. This movement is illustrated in Fig. 5B, which shows a side view of person 10. In solid lines, 51 shows an example for a comfortable range of movement, and extensions in dotted lines 53 show a corresponding maximum range of movement.

In other embodiments, other first and second movement ranges, e.g. passive and active maximum range of movement, may be used.

It should be noted that the order in which the first range and the second range are recorded is not fixed, i.e. the second range, for example comfortable range of movement, may be recorded before the first range, for example the maximum range of movement. In still other embodiments, the recording may be combined. For example, for the sideways movement of the upper torso illustrated in Fig. 5A, person 10 may first move to the right end of the maximum range of movement, for example press a button or otherwise confirm that the maximum range has been reached, then move back to the comfortable range, make another confirmation, then move to the end of the comfortable range on the other (left) side, confirm again, and the move to the maximum range of the left side. The above order may also be changed arbitrarily. In still other embodiments, for example movement to the left and to the right, in the example of Fig. 5A may be recorded separately.

As indicated by dots in Fig. 4, the number of different movement directions is not limited to two, but may be set to any arbitrary number depending on the types of exercises to be performed. For example, in addition to the movement directions illustrated in Figs 5A and 5B, first and second movement ranges for a movement of the upper torso in a front-back-direction, a movement of limbs etc. may be additionally or alternatively recorded.

As mentioned, the movements illustrated in Fig. 5A and 5B may for example be captured and stored using the sensor arrangement illustrated in Fig. 2. Also movement of other parts of the body may be captured using IMUs or similar sensors by placing IMUs on the corresponding body parts. However, other sensors may be used as well for recording the first and second ranges of movement at 30 and 31 in Fig. 3. For example, exoskeletons comprising motors and sensors may be used. For example, Fig. 6A schematically shows a person 10 equipped with exoskeleton devices 60 for the arms. Sensors 68, for example potentiometers, may be placed near the elbows, and when the elbows are bent and thus the exoskeleton devices 60 are bent at the elbows, the movement may be measured by sensors 61. Similar sensors may be placed at other joints, for example a shoulder joint. Such exoskeleton devices may additionally comprise motors or passive devices like spring-based devices to assist the movement of the arms of person 10 or even to fully move the arms of person 10. In this case, a range of motion may for example correspond to a reach of the arm.

Similar exoskeleton devices may for example be coupled with the legs of a person 10, as shown in Fig. 6B for exoskeleton devices 62. Again, at joints, for example at or near knee joints, sensors 63 may be placed. Person 10 may then for example walk or run, possibly assisted by motors of exoskeleton devices 62, on a treadmill 64 for performing the training. In such a case, a range of motion may for example correspond to a step length of a person.

Other sensor arrangements which may be used may comprise electromagnetic tracking devices, optical sensors in connection with optical markers, goniometers, angular sensors.

Returning now to Fig. 3, optionally at 32 a sensitivity is adjusted. Via this sensitivity, a mapping of movements of the person to movements of a movable object on a display may be influenced, as will be explained later in greater detail. The sensitivity may for example be adjusted using a slider represented on a screen. For example, in Fig. 4 a slider scale 41 with a slider 42 is shown. By moving slider 42 (for example by touch on a touchsreen or by clicking with a mouse pointer or the like), the sensitivity may be adjusted between a high sensitivity and a low sensitivity. With a high sensitivity, for example small movements may be sufficient to obtain large movements of a movable element on a screen to be discussed later, while with a low sensitivity larger body movements are required.

It should be noted that the sensitivity may also be adjusted in other ways than by using a sensitivity scale, for example by inputting a numerical sensitivity value or percentage or by using any other suitable input like for example a control dial.

In some embodiments, the sensitivity scale may be adapted to the first and second ranges of movement recorded at 30 and 31 in Fig. 3. For example, in some embodiments adjusting the sensitivity to a high value may enable a person who performs the training to perform the training (to be described later) with movements within the second range of movement, i.e. the comfortable range or exercise range. When moving to lower sensitivity, at some point a person is required to use his or her first movement range, i.e. the maximum movement range, to perform an exercise correctly. In some embodiments, this sensitivity may be marked by a marker like marker 47 of Fig. 4. It should be noted that in other embodiments an additional marker may be provided to mark a point where the exercises can just be performed within the second movement range. When going to even lower sensitivities than marker 47, this means that a person has to exceed the first range of movement earlier. Such exercises can then be useful to gradually help the person to increase his or her maximum range of movement.

It should be noted that in other embodiments the sensitivity scale may be independent from the first and second range of movement, or may depend only on one of the first and second movement ranges.

Returning now to Fig. 3, after the optional adjusting of the sensitivity in the embodiment of Fig. 3 a training begins which comprises the action described with reference to 33-36.

At 33, a movement of a person is measured using the sensors, and at 34 a feedback is output depending on the movement. In some embodiments, the feedback may comprise training instructions and an evaluation if the person training has complied with the training instructions. In other embodiments, a feedback may comprise outputting a game-like environment prompting the person to perform certain movements. Such a game-like environment may in particular comprise a movable part, the movement of which is controlled by movements of the person, and movement incentives prompting the user to perform certain movements to reach a certain training goal. Additionally, the feedback may comprise a score evaluating the person's performance.

In some embodiments, generating the feedback may comprise comparing measured movements with a normative database, for example comparative data obtained from measuring movements of a plurality of persons. Such a processing may be performed online in real time.

An example for such a feedback is depicted in Fig. 7, which schematically shows elements displayed on a display 70, which for example may correspond to display 11 of the apparatus shown in Fig. 1.

In the example of Fig. 7, an animal 71 is used as a movable object which is controlled by movements of a person training. For example, for a particular exercise animal 71 may be controlled by tilting movements of the pelvis of a person as depicted in Fig. 5B, a forward tilting resulting in an upward movement of animal 71 and a backward tilting resulting in a downward movement of animal 71. Furthermore, movement incentives 72-76 are displayed which in the example of Fig. 7 move from right to left with a certain speed. By moving animal 71 through tilting movements of the pelvis, animal 71 may "collect" movement incentives 72-76. The placement of movement incentives 72-76 is such that, when the training person manages to collect the movement incentives, the movements performed with the pelvis correspond to a desired training.

In some embodiments, the placement of movement incentives may depend on the first and second ranges of movement recorded at 30 and 31 in Fig. 3. For example, at a start of training the placement may be such that the person can fulfill the goal (for example collecting the movement incentives in the example of Fig. 7) by movements within the second range of movement, for example within the comfortable range. Then, in a second phase of training the placement of the movement incentives may be such that the person gradually has to go beyond the second range of movement until the first range of movement, i.e. the maximum range, is needed to fulfill the respective training goal, for example collecting the movement incentives in Fig. 7. In some cases, after the second phase a third phase may follow where an attempt is made to slowly increase the maximum range of movement, for example by placing the movement incentives such that a movement slightly beyond the maximum range is needed at least for some of the movement incentives. It should be noted that also in the second and third phases, some movement incentives may be placed such that they are easily reachable/obtainable by the person, for example by movements within the comfortable range. The transition between the phases may be automatically or controlled by a person supervising the training, for example a physiotherapist, or even controlled by the person training. In other embodiments, the movement incentive may be placed corresponding to the second range of movement, and for example after a predetermined training time or upon an input from the person training or another person like a physiotherapist, the second range of movement may be dynamically adjusted, for example increased, to gradually increase the person's second range of movement.

It should be noted that his placement of the movement incentives based on the first and second range of movement may be in addition to or alternatively to the regulation of the sensitivity mentioned earlier.

In Fig. 7, additionally a score 77 is displayed. The score may for example increase every time a movement incentive 72 is "collected".

It should be noted that additionally or alternatively to different placements of movement incentives in different phases, in Fig. 3 at 35, optionally the sensitivity may be adjusted during training. This may be performed manually by a user, for example by using a slider as explained with reference to Fig. 4, or automatically. For example, if it is detected that the person voluntarily moves beyond the second range of movement and therefore exceeds for example the comfortable range of movement, the sensitivity may be reduced to motivate the training person to perform even larger movements. In a similar manner, the sensitivity may be decreased when the person approaches the first range of movement. In still other embodiments, the sensitivity may decrease depending on time. As mentioned above, additionally or alternatively also the second range of movement may be adjusted, for example by a user input or even by re-capturing, i.e. by repeating step 31.

The action described with reference to 33, 34 and 35 in Fig. 3 are repeated until at 36 it is determined that the training is complete.

After the training is complete, at 37 overall results of the training, for example a final score, may be output and for example stored for future reference, for example for comparison with future trainings. After that, at 38 the method is ended.

It should be noted that the training described with reference to Fig. 7 serves only as an example, and virtually any visual, audible and/or tactile feedback may be used. Furthermore, also the score 77 may be determined depending on the first and second range of movement. For example, the score may increase when a certain percentage of the first range of movement, i.e. the maximum range of movement, is reached, for example 30%, 50% and 80%. For a final score, for example bonus points may be added when the first range of movement and/or the second range of movement is greater than the corresponding first range of movement and/or second range of movement in a previous training.

Also, for example the preciseness of the movement, for example in Fig. 7 how precise the movable object 71 is controlled to reach the movement incentives 72-76, may be evaluated.

It should be noted that other kinds of exercises in a game-like environment may be provided as well. For example, in some training exercises a stabilization of the body, i.e. the maintaining of the body in a certain position, may be required. In this case, as a movable object for example a bridge may be depicted, and as long as the position is held, movement incentives may go over the bridge. When the position is left, for example within a certain range around the target position is left, the bridge opens and the incentives fall down. In still other embodiments, for example body movements as shown in Fig. 5A may be used to control an angle of a slide to sort movement incentives into certain baskets. Also in this case, a first and second movement range may be used as described above.

Furthermore, while the exercises described with reference to Fig. 7 concern exercises in a single movement direction or movement plane, also exercises requiring a movement in a three-dimensional movement space and a corporation of a plurality of joints are equally possible. In this case, as mentioned above, also the first and second ranges of movement may be three-dimensional ranges in more than one direction.

In the above embodiments, ranges of movement have been used as example for first and second movement-related parameter spaces. In other embodiments, other types of parameter spaces may be captured. For example, in an embodiment the first and second parameter spaces may correspond to first and second forces exerted by a body part of a person in an isometric manner, i.e. without actual movement, for example by pressing against a pressure sensor like a piezoelectric sensor or other pressure sensor. For example, in this case the first movement-related parameter space may comprise a maximum force a person is able to exert, and the second movement-related parameter space may comprise an exercise force. Such forces may for example be gripping forces, i.e. forces exerted by trying to perform a gripping movement of fingers of the hand against a fixed resistance, such that the exercise becomes isometric. In such a case, during a training feedback may then be based on an actual force exerted, which is another example of a movement-related parameter besides an actual movement.

As can be seen from the above, a plurality of modifications and variations are possible within leaving the scope of the present invention. Therefore, the above-described embodiments are to be regarded as examples only and are not to be construed as limiting the scope of the application.

## Claims

1. A training apparatus, comprising:
a sensor arrangement (12; 20A-C; 61, 63) configured to capture a movement-related parameter of a person (10),
a storage (14) and
an evaluation unit (13) coupled to the sensor arrangement (12; 20A-20C; 61; 63) and the storage (14), the evaluation unit (13) being configured to capture a first movement-related parameter space of the person (10) via the sensor arrangement (12; 20A-C; 61; 63), to capture a second movement-related parameter space of the person (10) via the sensor arrangement (12; 20A-C; 61; 63), the second movement-related parameter space being at least partially within the first movement-related parameter space, and to store the first movement-related parameter space and the second movement-related parameter space in said storage (14).

2. The training apparatus of claim 1, wherein the first movement-related parameter space comprises a first range of movement of the person (10) and the second movement-related parameter space comprises a second range of movement of the person (10), the second range of movement being within the first range of movement.

3. The training apparatus of claim 1 or 2, wherein the apparatus is configured to capture the first movement-related parameter space and to capture the second movement-related parameter space in an initialization phase prior to a training phase.

4. The training apparatus of claim 3, wherein the apparatus is configured to modify said second movement-related parameter space during said training phase.

5. The training apparatus according to claim 3 or 4, further comprising an output device (11), wherein said evaluation unit (13) is further configured to, during said training phase, output a feedback via said output device based on a movement-related parameter of the person (10) captured by said sensor arrangement (12; 20A-C; 61; 63).

6. The training apparatus of claim 5, wherein said output device comprises a display (11), wherein said feedback comprises a movable object (71) on said display, movements of the movable object (71) depending on a movement-related parameter of the person (10) captured by the sensor arrangement (12; 20A-C; 61; 63).

7. The training apparatus of claim 6, further comprising an input (15) configured to receive a sensitivity, wherein a mapping of the movement-related parameter of the person (10) to a movement of the movable object (71) depends on said sensitivity.

8. The apparatus of claim 7, wherein said sensitivity is scaled based on the first movement-related parameter space and/or based on the second movement-related parameter space.

9. The training apparatus of any one of claims 6-8, wherein said feedback further comprises movement incentives (72-76) on said screen, wherein a placement of the movement incentives (72-76) depends on said first movement-related parameter space and/or said second movement-related parameter space.

10. The training apparatus of any one of claims 5-9, wherein said feedback depends on the first movement-related parameter space and/or the second movement-related parameter space.

11. The training apparatus of any one of claims 5-10, wherein the evaluation unit (13) is configured to compare a movement-related parameter of the person (10) during the training phase with the first movement-related parameter space and/or with the second movement-related parameter space.

12. The training apparatus of claim 10 and of claim 11, wherein said evaluation unit (13) is configured to adapt the feedback based on the comparison.

13. The training apparatus of claim 11 or claim 12, wherein said feedback comprises a score, and wherein said score is calculated depending on said comparison.

14. The training apparatus of any one of claims 1-13, wherein said evaluation unit (13) is configured to compare said first movement-related parameter space and/or said second movement-related parameter space with a first movement-related parameter space and/or a second movement-related parameter space stored during a previous training session.

15. The training apparatus of any one of claims 1-14, wherein said sensor arrangement (12) comprises one or more sensors selected from the group consisting of an inertial measurement unit, an acceleration sensor, a sensor arranged in an exoskeleton, an optical sensor, an electromagnetic tracking device, a sensor using an optical marker, a goniometer, a linear potentiometer and a pressure sensor.
